# EUROPÄISCHE PATENTANMELDUNG

(11) **EP 0 639 570 A1**
(43) Veröffentlichungstag der Anmeldung: **22.02.1995**
(21) Anmeldenummer: 94111824.2
(22) Anmeldetag: 29.07.1994
(51) Int. Cl.: C07D 215/48

(54) **Verfahren zur Herstellung von aromatischen Carbonsäuren**

(30) Priorität: 05.08.1993 DE 4326288
(71) Anmelder: BASF Aktiengesellschaft, D-67063 Ludwigshafen (DE)
(72) Erfinder: Hagen, Dr. Helmut, D-67227 Frankenthal (DE); Guldner, Dr. Andreas, D-69469 Weinheim (DE)

(57) **Zusammenfassung**

Herstellung von aromatischen Carbonsäuren der Formel I

Ar(COOH)ₙ I

(Ar = iso- oder heteroaromatischer Rest, n = 1 bis 4), durch Oxidation einer Verbindung II

Ar(CH₃)ₙ II

mit einer oxidierenden Stickstoff/Sauerstoff-Verbindung (III) in Gegenwart von Schwefelsäure und einer Vanadium-Verbindung (IV) bei 120 bis 180°C in flüssiger Phase, indem im Reaktionsgemisch pro Mol II 20 bis 100 Mol-% der Vanadium-Verbindung (IV) für die Oxidation zur Verfügung stehen.

## Beschreibung

Die vorliegende Erfindung betrifft ein verbessertes Verfahren zur Herstellung von aromatischen Carbonsäuren der Formel I

Ar(COOH)ₙ I

in der Ar einen iso- oder heteroaromatischen Rest bedeutet, der unsubstituiert sein kann oder inerte Substituenten tragen kann, und in der n für 1 bis 4 steht, durch Oxidation einer Verbindung II

Ar(CH₃)ₙ II

mit einer oxidierenden Stickstoff/Sauerstoff-Verbindung (III) in Gegenwart von Schwefelsäure und einer Vanadium-Verbindung (IV) bei 120 bis 180°C in flüssiger Phase.

Die Herstellung von aromatischen Carbonsäuren durch direkte Oxidation von methylsubstituierten Aromaten ist in zahlreichen Ausgestaltungen allgemein bekannt. Im Hinblick auf ihre Bedeutung als Zwischenprodukte für zahlreiche Farbstoffe, Arzneimittel und Pflanzenschutzmittel sind die heteroaromatischen Carbonsäuren hierbei von besonderem Interesse.

Insbesondere gilt dies für Chinolincarbonsäuren, die wichtige Zwischenprodukte für die Synthese von Pharmazeutika darstellen. Als Wirkstoffe im Pflanzenschutz werden die aus der EP-B 277 631 bekannten halogensubstituierten Chinolin-8-carbonsäuren angewendet.

Aus J. Am. Chem. Soc. 68, 2721 (1946) geht hervor, daß Verbindungen wie Chinolin-5-, -6- und -7-carbonsäure sowie 2-Phenylchinolin-5-carbonsäure als Ausgangsverbindungen für die Herstellung von - gegen Malaria wirksamen - α-(2-Piperidyl)-chinolinmethanolen dienen. Die Synthese der Chinolincarbonsäuren erfolgt durch Oxidation der entsprechenden Methylchinoline, wobei man als Oxidationsmittel Chromtrioxid und Arsensäure verwendet, jedoch sind die Ausbeuten und Selektivitäten unbefriedigend, ganz abgesehen davon, daß die Verwendung dieser Oxidationsmittel erhebliche verfahrenstechnische Probleme aufwirft.

In der EP-A 282 778 werden die Herstellung von 7-Chlorchinolin-8-carbonsäure sowie ihrer in 3-Position durch Chlor oder Methyl substituierten Derivate beschrieben, welche man durch direkte Oxidation des entsprechenden 8-Methylchinolins mit Salpetersäure oder mit Stickstoffdioxid in Gegenwart von Schwefelsäure und Vanadinpentoxid als Katalysator erhält. Da unter den hier gewählten Verfahrensbedingungen Nitrochinolincarbonsäuren und kernnitrierte Methylchinoline als Nebenprodukte entstehen, läuft diese Umsetzung nicht mit ausreichend hohen Ausbeuten und Selektivitäten ab.

Der vorliegenden Erfindung lag daher die Aufgabe zugrunde, den geschilderten Mängeln abzuhelfen und die aromatischen Carbonsäuren I einfacher und wirtschaftlicher zugänglich zu machen als bisher.

Demgemäß wurde ein Verfahren zur Herstellung von aromatischen Carbonsäuren der Formel I

Ar(COOH)ₙ I

in der Ar einen iso- oder heteroaromatischen Rest bedeutet, der unsubstituiert sein kann oder inerte Substituenten tragen kann, und in der n für 1 bis 4 steht, durch Oxidation einer Verbindung II

Ar(CH₃)ₙ II

mit einer oxidierenden Stickstoff/Sauerstoff-Verbindung (III) in Gegenwart von Schwefelsäure und einer Vanadium-Verbindung (IV) bei 120 bis 180°C in flüssiger Phase gefunden, welches dadurch gekennzeichnet ist, daß im Reaktionsgemisch pro Mol II 20 bis 100 Mol-% der Vanadium-Verbindung (IV) für die Oxidation zur Verfügung stehen.

Ferner wurde gefunden, daß dieses Verfahren im Falle von heteroaromatischen Carbonsäuren besonders gut gelingt, und daß es ganz besonders gut gelingt, wenn man es zur Herstellung von Chinolincarbonsäuren der Formel V 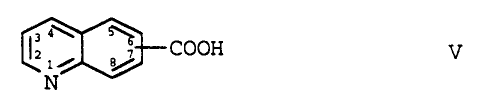
insbesondere von Chinolin-6-carbonsäure anwendet.

Als Reste Ar in den Verfahrensprodukten I bzw. Ausgangsverbindungen II eignen sich beispielsweise diejenigen, die sich von
- isoaromatischen Verbindungen wie Benzol, Naphthalin, Anthracen und Phenanthren sowie kondensierten polycyclischen Aromaten wie Chrysen und Benzanthracen und von
- heteroaromatischen Verbindungen wie Furan, Thiophen, Pyrrol, Oxazol, Isoxazol, Oxadiazolen, Thiazol, Isothiazol, Thiadiazolen, Pyrazol, Imidazol, Triazolen, Pyridin, Pyridazin, Pyrimidin und Pyrazin als Stammkörper
ableiten.

Insbesondere sind solche Heteroaromaten geeignet, welche anellierte Ringe tragen wie vor allem im Chinolin und Isochinolin sowie ferner im Benzofuran, Isobenzofuran, Benzothiophen, Indol, Isoindol, Benzisoxazol, Benzoxazol, Benzisothiazol, Benzthiazol, Indazol, Purin, Chinoxalin, Chinazolin, Phthalazin, Indolin, Isoindolin, Chroman und im Isochroman.

In den entsprechenden Ausgangsverbindungen II sind 1 bis 4 Methylgruppen an den Rest Ar gebunden, wobei die Anzahl der Methylgruppen vorzugsweise 1 und 2, insbesondere 1 beträgt. Für den Fall, daß Ar für einen heteroaromatischen Stammkörper mit einem anellierten Ring steht, ist vorzugsweise mindestens eine Methylgruppe an den anellierten Ring gebunden.

Die übrigen C-Atome können beliebige inerte Substituenten, d.h. nicht selber oxidierbare Reste tragen, beispielsweise Fluor, Chlor, Brom, Jod, Nitro, Carboxyl und Hydroxy sowie Aryl wie Phenyl, α-Naphthyl und β-Naphthyl und Aralkyl wie Benzyl sowie 1- und 2-Phenylethyl.

Auch C₁-C₆-Alkylgruppen sowie
- Cycloalkyl wie Cyclopentyl und Cyclohexyl,
- cycloalkylsubstituiertes Alkyl wie 1- und 2-Cyclohexylethyl,
- Oxacycloalkyl wie 2- und 3-Oxacyclopentyl, 2-, 3- und 4-Oxacyclohexyl sowie
- C₁-C₄-Alkoxy wie Methoxy, Ethoxy, n-Propyloxy, Isopropyloxy, n-Butyloxy, Isobutyloxy, sec.-Butyloxy und tert.-Butyloxy kommen unter Umständen in Betracht, sofern sie unter den Reaktionsbedingungen nicht oder nicht wesentlich angegriffen werden. Diese Substituenten verhalten sich in anellierten heteroaromatischen Ringen häufig inert, insbesondere wenn sie an den heteroaromatischen Stammkörper gebunden sind.

Außerdem können die inerten Substituenten selber substituiert sein, beispielsweise durch Halogen, Nitro, Cyano und Alkoxy.

Bevorzugte Ausgangsverbindungen II sind methylsubstituierte Heteroaromaten. Im Hinblick auf die erwünschten Verfahrensprodukte Chinolincarbonsäuren V sind die Methylchinoline der Formel II' 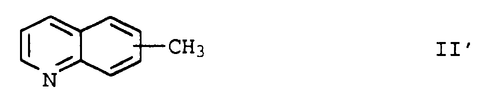
besonders bevorzugt, wobei hinsichtlich des Produktes Chinolin-6-carbonsäure die Verbindung 6-Methylchinolin ganz besonders bevorzugt ist.

Die Ausgangsverbindungen II sind entweder kommerziell erhältlich oder können nach bekannten Verfahren hergestellt werden (J. Am. Chem. Soc. 66, 396 (1944)).

Die im Hinblick auf die Verfahrensprodukte Chinolincarbonsäuren besonders wichtigen Ausgangsverbindungen 5-, 6-, 7- und 8-Methylchinolin sind aus J. Chem. Soc. 3645 (1962), Monatsh. 2, 139 (1881) und Brit. Pat. 198 462 bekannt und lassen sich hiernach herstellen.

Die Verfahren zur Herstellung von I werden nach den üblichen Techniken in flüssiger Phase sowohl auf diskontinuierliche als auch auf kontinuierliche Weise durchgeführt. Bei der diskontinuierlichen Arbeitsweise erfolgt die Oxidation von II zu I in einem Rührkessel durch Mischen sämtlicher Reaktionsteilnehmer. Dabei kann es sich zur Erhöhung der Selektivität bezüglich I empfehlen, den Umsatz von II auf etwa 50, vorzugsweise 80 % zu begrenzen. In der Regel erzielt man aber auch bei vollständigem Umsatz gute Selektivitäten.

Bei der kontinuierlichen Verfahrensgestaltung kann die Reaktion beispielsweise in einem Rührkessel mit nachgeschaltetem Rohrreaktor, in einer Rührkesselkaskade oder in einem Rohrreaktor stattfinden.

Als Oxidationsmittel dienen oxidierende Stickstoff/Sauerstoff-Verbindungen (III), wobei vor allem Salpetersäure geeignet ist. Sie kann sowohl in konzentrierter Form als auch mit Wasser verdünnt eingesetzt werden. Weiterhin kommen Stickstoff/Sauerstoff-Verbindungen wie Distickstoffpentoxid sowie besonders Stickstoffdioxid und sein Dimeres Distickstofftetroxid in Betracht. Außerdem ist es möglich, Sauerstoff sowie die bei der Umsetzung von II zu I entstehenden stickstoffoxidhaltigen Reaktionsabgase zusammen mit III als Oxidationsmittel zu verwenden. Sauerstoff kann sowohl in reiner Form als auch in Form von Luft eingesetzt werden. Im allgemeinen bewirkt er eine Reaktionsbeschleunigung.

Für die Umsetzung von II zu I werden zweckmäßigerweise 2 bis 8, besonders 5 bis 7 mol III pro Mol Ausgangssubstanz II verwendet.

Üblicherweise arbeitet man unter Atmosphärendruck. Bei Verwendung gasförmiger Stickstoff/Sauerstoff-Verbindungen empfiehlt es sich, die Umsetzung bei einem leicht erhöhten Druck bis ca. 10 bar durchzuführen.

Als Oxidationskatalysator werden Verbindungen (IV) des fünfwertigen Vanadiums, vorzugsweise solche, die in Schwefelsäure und Gemischen aus Schwefelsäure und Salpetersäure löslich sind, verwendet. Neben dem Sulfat, Nitrat und Acetat kommen insbesondere Vanadinpentoxid und Ammoniumpolyvanadat zum Einsatz. Ein wesentliches Merkmal der Erfindung ist es, höhere Mengen an IV, als üblicherweise für katalytische Reaktionen erforderlich sind, zu verwenden. Unter höheren Mengen ist zu verstehen, daß bei der Herstellung von I pro kg II 0,1 bis 0,6 kg, insbesondere 0,2 bis 0,5 kg der Vanadium-Verbindung (IV) eingesetzt werden. Bei Verwendung geringerer Mengen an IV ist im allgemeinen ein Absinken der Selektivität infolge von Nebenreaktionen wie Nitrierung zu beobachten.

Als Reaktionsmedium dient Schwefelsäure, welche in Konzentrationen von ca. 50 bis 90 %, insbesondere 70 bis 85 % verwendet werden kann. In der Regel ist es zweckmäßig, daß die Reaktion in einer Menge von 2 bis 10 kg Schwefelsäure pro kg II durchgeführt wird.

Die Reaktionstemperatur liegt bei 120 bis 180°C, vorzugsweise bei 130 bis 170°C.

Im übrigen erfolgt die Umsetzung in an sich bekannter Weise, indem man II in Schwefelsäure löst, diese Lösung mit der Vanadium-Verbindung (IV) versetzt und anschließend III hinzufügt. Für die Aufarbeitung wird die Reaktionsmischung in der Regel abgekühlt, mit Wasser verdünnt und mit einer Base wie Natriumhydroxid auf einen pH-Wert von ungefähr 0,5 bis 5,0 gebracht, wobei das Verfahrensprodukt I ausfällt. Dieses kann dann durch übliche Methoden wie Filtration und Umkristallisation isoliert und gereinigt werden.

Im Falle der heteroaromatischen Verfahrensprodukte, insbesondere der Chinolincarbonsäuren V, können diese bereits sofort nach dem Verdünnen mit Wasser in Form ihrer schwefelsauren Salze anfallen. Zweckmäßigerweise lassen sie sich durch Filtration von der Reaktionslösung abtrennen. Die Salze können dann auf übliche Weise in wäßriger Lösung mittels einer Lauge wie Natronlauge in die entsprechende Base überführt werden.

Ferner ist es ein Vorteil, daß die bei der Aufarbeitung anfallende Schwefelsäure und das die Vanadium-Verbindung (IV) enthaltende Filtrat mehrfach verwendet werden kann.

Aromatische Carbonsäuren I, die nach den erfindungsgemäßen Verfahren hergestellt werden, sind wertvolle Produkte für eine große Anzahl von Anwendungen. Besonders sind die Chinolincarbonsäuren V von Interesse, da sie wichtige Zwischenprodukte für Arzneimittel sind. Ganz besondere Bedeutung haben die erfindungsgemäßen Verfahren für die Herstellung von Chinolin-6-carbonsäure.

### Herstellung von Chinolin-6-carbonsäure

### Beispiel 1

Eine Lösung von 106 g (0,74 mol) 6-Methylchinolin in 600 g 60 %iger Schwefelsäure wurde mit 37 g (0,2 mol) Vanadinpentoxid versetzt und auf eine Temperatur von ca. 133°C erhitzt. Innerhalb von 8 Stunden wurden hierzu 619 g (6,4 mol) 65 %ige Salpetersäure eingeleitet. Gleichzeitig wurden 380 ml 35 %ige Salpetersäure abdestilliert. Nach Abkühlung auf 50°C wurde die Reaktionsmischung mit 420 ml Wasser verdünnt und noch weitere 10 Stunden bei 25°C gerührt. Anschließend wurden 114 g des schwefelsauren Salzes der Chinolin-6-carbonsäure abfiltriert.

Das dabei anfallende Filtrat, enthaltend verdünnte Schwefelsäure und gelöstes Vanadinpentoxid, wurde durch Einengung auf die ursprüngliche Konzentration von 60 %iger Schwefelsäure gebracht. Es wurden 570 g einer Lösung von Vanadinpentoxid in 60 %iger Schwefelsäure erhalten.

Das schwefelsaure Salz der Chinolin-6-carbonsäure wurde in 800 ml Wasser mit konzentrierter Natronlauge bei 40°C bis zu einem pH-Wert von ca. 3,5 bis 4 unter Rühren in die Chinolin-6-carbonsäure überführt. Nach Abkühlung dieser Lösung wurde das ausgefallene Produkt abfiltriert.
Ausbeute: 83 g Chinolin-6-carbonsäure (65 % d. Th.)

### Beispiel 2

In einer Mischung aus 98 g konzentrierter Schwefelsäure und 570 g vanadinpentoxidhaltiger 60 %iger Schwefelsäure (eingeengtes Filtrat gemäß Beispiel 1) wurden 106 g (0,74 mol) 6-Methylchinolin gelöst. Nach Erhitzen auf 135°C wurden innerhalb von 7 Stunden 542 g (5,6 mol) 65 %ige Salpetersäure eingeleitet. Die Durchführung und Produktaufarbeitung erfolgte gemäß Beispiel 1.
Ausbeute: 95 g Chinolin-6-carbonsäure (74 %)

### Beispiel 3

Die Herstellung von Chinolin-6-carbonsäure erfolgte entsprechend Beispiel 1. Während der Zugabe der 65 %igen Salpetersäure wurde außerdem Luft in einer Menge von 5 l pro Stunde eingeleitet.
Ausbeute: 97 g (76 %)

### Beispiel 4

Die Herstellung von Chinolin-6-carbonsäure erfolgte entsprechend Beispiel 1. Während der Zugabe der 65 %igen Salpetersäure wurde das bei der Umsetzung entstehende stickoxidhaltige Reaktionsabgas zurückgeführt und in die Reaktionslösung eingeleitet.
Ausbeute: 97 g (76 %)

### Beispiel 5

Eine Lösung von 106 g (0,74 mol) 6-Methylchinolin in 600 g 60 %iger Schwefelsäure wurde mit 37 g (0,2 mol) Vanadinpentoxid versetzt und auf eine Temperatur von ca 130°C erhitzt. Anschließend wurde Stickstoffdioxid bis zu einem Überdruck von 0,2 bar eingeleitet. Danach wurde kontinuierlich Sauerstoff zugeführt, so daß ein konstanter Überdruck von 0,5 bar eingehalten wurde. Nachdem nach 9 Stunden kein Sauerstoff mehr verbraucht wurde, wurde die Reaktionsmischung entsprechend dem Beispiel 1 aufgearbeitet.
Ausbeute: 90 g Chinolin-6-carbonsäure (70 %)

## Patentansprüche

1. Verfahren zur Herstellung von aromatischen Carbonsäuren der Formel I
Ar(COOH)ₙ I
in der Ar einen iso- oder heteroaromatischen Rest bedeutet, der unsubstituiert sein kann oder inerte Substituenten tragen kann, und in der n für 1 bis 4 steht, durch Oxidation einer Verbindung II
Ar(CH₃)ₙ II
mit einer oxidierenden Stickstoff/Sauerstoff-Verbindung (III) in Gegenwart von Schwefelsäure und einer Vanadium-Verbindung (IV) bei 120 bis 180°C in flüssiger Phase, dadurch gekennzeichnet, daß im Reaktionsgemisch pro Mol II 20 bis 100 Mol-% der Vanadium-Verbindung (IV) für die Oxidation zur Verfügung stehen.

2. Verfahren zur Herstellung von aromatischen Carbonsäuren (I) nach Anspruch 1, dadurch gekennzeichnet, daß man es auf die Herstellung von heteroaromatischen Carbonsäuren anwendet.

3. Verfahren zur Herstellung von aromatischen Carbonsäuren (I) nach Anspruch 2, dadurch gekennzeichnet, daß man es auf die Herstellung von Chinolincarbonsäuren der Formel V 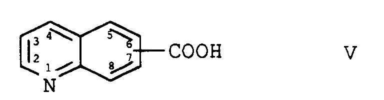 anwendet.

4. Verfahren zur Herstellung von aromatischen Carbonsäuren (I) nach Anspruch 3, dadurch gekennzeichnet, daß man es auf die Herstellung von Chinolin-6-carbonsäure anwendet.
